(19) Europäisches Patentamt
European Patent Office
Office européen des brevets

(11) Veröffentlichungsnummer: **0 302 993 B1**

(12) # EUROPÄISCHE PATENTSCHRIFT

(45) Veröffentlichungstag der Patentschrift: **06.05.92**

(51) Int. Cl.[5]: **C07C 35/205**

(21) Anmeldenummer: **88104540.5**

(22) Anmeldetag: **22.03.88**

(54) **Methylcyclododecatrien-1-ole, Verfahren zu ihrer Herstellung und ihre Verwendung.**

(30) Priorität: **02.04.87 DE 3711157**

(43) Veröffentlichungstag der Anmeldung:
**15.02.89 Patentblatt 89/07**

(45) Bekanntmachung des Hinweises auf die Patenterteilung:
**06.05.92 Patentblatt 92/19**

(84) Benannte Vertragsstaaten:
**CH DE FR GB IT LI NL**

(56) Entgegenhaltungen:
**FR-A- 2 444 656**
**GB-A- 1 152 932**
**US-A- 3 723 478**
**US-A- 3 816 349**

**FIESER AND FIESER'S "Reagents for organic synthesis", Band 9, 1980, Band 9, 1981, Wiley-Interscience Publication, New York, US**

**PATENT ABSTRACTS OF JAPAN, Band 6, Nr. 197 (C-128)[1075], 6. Oktober 1982; & JP - A - 57 106 633**

(73) Patentinhaber: **HAARMANN & REIMER GMBH**
**Postfach 138**
**W-3450 Holzminden(DE)**

(72) Erfinder: **Nienhaus, Jürgen, Dr.**
**Eckenbrecherstrasse 20**
**W-3450 Holzminden(DE)**
Erfinder: **Hopp, Rudolf, Dr.**
**Auf dem Gehrenkamp 28**
**W-3450 Holzminden(DE)**
Erfinder: **Göttsch, Wilhelm**
**Eichenhang 12**
**W-3454 Bevern(DE)**
Erfinder: **Oelkers, Egon**
**Rotdornstrasse 15**
**W-3454 Bevern(DE)**

(74) Vertreter: **Zobel, Manfred, Dr. et al**
**c/o BAYER AG Konzernverwaltung RP Patente Konzern**
**W-5090 Leverkusen 1 Bayerwerk(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.18/2.0)

## Beschreibung

Die Erfindung betrifft neue Methylcyclododecatrien-1-ole, ein Verfahren zu ihrer Herstellung und ihre Verwendung als Riechstoff.

Aus der US-PS 3 723 478 ist der gesättigte Alkohol 2,6,9-Trimethyl-cyclododecan-1-ol als Zwischenprodukt für die Herstellung des gesättigten Ketons 2,6,9-Trimethyl-cyclododecan-1-on bekannt; zu den sensorischen Eigenschaften des Alkohols werden jedoch keinerlei Angaben gemacht. In der US-PS 3 845 078 wird beschrieben, daß das Gemisch der geometrischen Isomeren von 1,5,9-Trimethylcyclododeca-4,8-dien-1-ol einen beständigen holzartigen Vetiverduft aufweist.

Aus der FR-A 2 444 656 sind 1-Alkoxy-1,4,8-trimethyl-cyclododecadi-4,8-ene und ihre Verwendung als Riechstoffe bekannt. Sie sollen einen holzigen Geruch ähnlich Patchouli besitzen.

Überraschenderweise wurde nun gefunden, daß Verbindungen der Formel

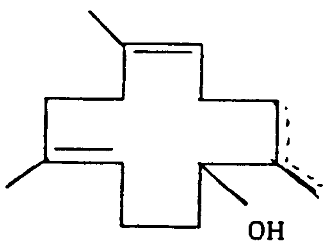

(I),

in der

die gestrichelten Linien die beiden möglichen Positionen der dritten Doppelbindung darstellen und die endocyclischen Doppelbindungen cis- und transkonfiguriert sein können,

im Unterschied zu den bekannten 2,6,9-Trimethylcyclododecan-Derivaten eine feine Holznote mit den Nuancen Ambra, Moschus und Sandel und damit äußerst wertvolle, in der Parfümerie gesuchte Geruchseigenschaften aufweisen.

Die Erfindung betrifft daher Methylcyclododecatrien-1-ole der Formel I.

Bei den erfindungsgemäßen Verbindungen der Formel I handelt es sich um das 2,6,9-Trimethyl-cyclododecatri-2,5,9-en-1-ol Ia und sein Isomer 2-Methylen-6,9-dimethylcyclododecadi-5,9-en-1-ol.Ib.

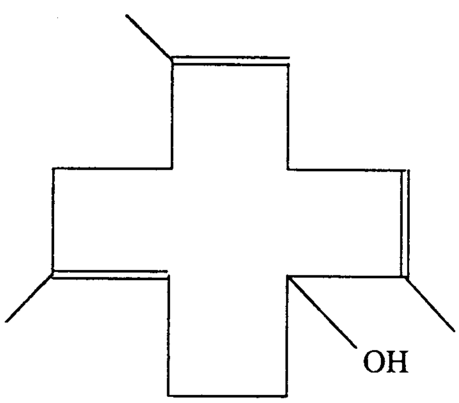

Ia

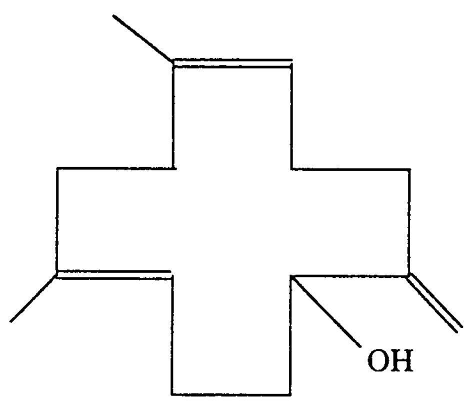

Ib

Die beiden Verbindungen der Formel I werden dadurch erhalten, daß man 1,5,10-Trimethyl-cyclododeca-1,5,9-trien, eine im Handel erhältliche Verbindung, in an sich bekannter Weise mit einer Persäure monoepoxidiert und das erhaltene Monoepoxid 1,6,9-Trimethyl-1,2-epoxy-cyclododeca-5,9-dien in Gegenwart von Aluminiumisopropylat erhitzt. Durch das Erhitzen des Epoxydierungsproduktes in Gegenwart von Aluminiumisopropylat wird eine praktisch einsinnig verlaufende Öffnung des Epoxyringes durch Isomerisierung erreicht.

Die Erfindung betrifft daher ein Verfahren zur Herstellung der Verbindungen der Formel I, daß dadurch gekennzeichnet ist, daß man 1,5,10-Trimethylcyclododeca-1,5,9-trien in an sich bekannter Weise monoepoxydiert und das erhaltene 1,6,9-Trimethyl-1,2-epoxy-cyclododeca-5,9-dien durch Erhitzen in Gegenwart von Aluminiumisopropylat unter Öffnung der Epoxygruppe isomerisiert.

Die Epoxidierung des Ausgangstriens wird mit Persäuren wie Perbenzoesäure, Monoperphthalsäure oder vorzugsweise Peressigsäure in inerten Lösungsmitteln wie Chloroform, Methylenchlorid, Benzol oder vorzugsweise Toluol nach den üblichen, in der Literatur, z.B. in der US-P 3 723 478, beschriebenen Verfahren, durchgeführt. Die bei einem etwa 10 %igen Überschuß der Persäure, bezogen auf das cyclische Trien, entstehenden geringen Mengen an Diepoxid lassen sich leicht destillativ entfernen.

Die Isomerisierung des Monoepoxides kann in Gegenwart von inerten Lösungsmitteln, z.B. Toluol, durchgeführt werden; vorzugsweise wird jedoch ohne Lösungsmittel gearbeitet. Das Aluminiumisopropylat wird in einer Menge von 0,5 bis 5 Mol-%, vorzugsweise 1 bis 3 Mol-%, bezogen auf das Monoepoxid angewendet. Die Reaktion wird bei Temperaturen von 120 bis 180°C, vorzugsweise 140 bis 160°C, vorgenommen; vorzugsweise wird bei z.B. einem Unterdruck von 3 mbar bis 7 mbar gearbeitet.

Die Erfindung betrifft weiterhin die Verwendung der erfindungsgemäßen Verbindungen der Formel I als Riechstoff. Sie werden in Kombination mit anderen, an sich bekannten Riechstoffen, wie sie z.B. im Arctander, Perfume and Flavor Chemicals, Montclair, N.J. (USA), 1969 beschrieben sind, und etherischen Ölen, wie sie im Arctander, Perfume and Flavor Materials of natural Origin, Elisabeth, N.J. (USA), 1960 beschrieben sind, angewendet. Sie verleihen diesen Duftkompositionen einen dezenten, langanhaltenden Holzduft mit gut abrundenden und fixierenden Eigenschaften. Im allgemeinen werden sie in einer Menge von 1 bis 80 Gew.-%, vorzugsweis 3 bis 25 Gew.-%, bezogen auf die Duftkomposition, angewendet.

Die unter Verwendung der erfindungsgemäßen Verbindungen der Formel I hergestellten Duftkompositionen eignen sich hervorragend für die Parfümierung von Fertigprodukten des Aerosol-, Waschmittel- und des Chemisch-Technischen-Sektors, insbesondere aber des Feinparfümerie- und Kosmetiksektors. Die Herstellung der Duftkompositonen und parfümierten Produkte erfolgt in üblicher Weise, beispielsweise durch Zusammengeben der Komponenten.

Beispiel 1

Zur Mischung aus 1.836 g (9 Mol) 1,5,10-Trimethyl-cyclododeca-1,5,9-trien, 171 g (1,62 Mol) Soda und 2.700 g Toluol werden unter Rühren bei 10 bis 20°C innerhalb von 2 Stunden 1.938 g (10,2 Mol) Peressigsäure (40 %ig) zudosiert. Das Reaktionsgemisch wird 3 Stunden bei 10 bis 20°C gerührt und anschließend mit 1.500 g Wasser verdünnt. Die organische Phase wird abgetrennt, neutral gewaschen und vom überschüssigen Peroxid durch Zugabe von Eisen-II-ammoniumsulfat befreit. Dann wird das Lösungsmittel entfernt und der Rückstand destilliert. Die Ausbeute beträgt 1.444 g (= 72,9 % der Theorie) 1,6,9-Trimethyl-1,2-epoxy-cyclododeca-5,9-dien, Kp: 101°C/1 mbar.

1.444 g (6,55 Mol) des so erhaltenen Monoepoxides werden nach Zugabe von 25 g (0,12 Mol) Aluminiumisopropylat auf 140 bis 145°C erhitzt, wobei gleichzeitig ein Vakuum von 5 mbar eingestellt wird. Nach 3-stündigem Erhitzen auf Rückflußtemperatur, wird das Reaktionsgemisch zunächst einer Grob- und dann einer Feindestillation unterworfen. Es werden 1.209 g (83,7 % der Theorie) des Isomerengemisches aus 2,6,9-Trimethyl-cyclododecatri-2,5,9-en-1-ol und 2-Methylen-6,9-dimethyl-cyclododecandi-5,9-en-1-ol, Kp: 111°C/0,4 mbar, erhalten.

Beispiel 2

Ein Parfümöl mit einer Damennote wird durch Mischen der folgenden Komponenten hergestellt:

| | |
|---|---|
| Alpha-Iso-Methyljonon | 100 |
| Phenylethylalkohol | 70 |
| Hexenylsalicylat | 50 |
| Benzylsalicylat | 40 |
| Cyclopentadecanolid | 25 |
| Jasmin-Base | 50 |
| Rosenbase Typ de Mai | 40 |
| Styrolylacetat | 20 |
| Menthylacetat | 80 |
| Acetanisol | 5 |
| Dimethylbenzylcarbinylacetat | 40 |
| Octincarbonsäuremethylester 10 % in Dipropylenglycol | 5 |
| Hexylzimtaldehyd alpha | 60 |
| 4-(4-Hydroxy-4-methylpentyl)-3-cyclohexen-1-carboxaldehyd | 40 |
| Isoeugenolmethylether | 80 |
| Moschus-Keton | 15 |
| 2-tert.-Butylcyclohexylacetat | 30 |
| Allylamylglycolat | 3 |
| Damascon alpha | 2 |
| Nonadienal 1 % in Diethylphthalat | 6 |
| Ylang-Ylangöl | 40 |
| Undec-8-en-1-al | 2 |
| 2,6,9-Trimethyl-cyclododecatri-2,5,9-en-1-ol + Isomer | 120 |
| Dipropylenglycol | 77 |
| | 1.000 |

Beispiel 3

Ein Parfümöl mit einer Herrennote wird durch Mischen der folgenden Komponenten hergestellt:

| | |
|---|---|
| Bergamottöl afrik. synth. | 100 |
| Rosmarinöl | 20 |
| Limetteöl westind. dest. | 20 |
| Basilicumöl | 10 |
| Beifußöl | 10 |
| Kiefernnadelöl | 20 |
| Cyclohexylpropionsäureallylester | 4 |
| Prenylacetat | 1 |
| Hydroxycitronellal | 50 |
| Hexylzimtaldehyd alpha | 40 |
| 4-(4-Hydroxy-4-methylpentyl)-3-cyclohexen-1-carboxaldehyd | 30 |
| Dihydromyrcenol | 40 |
| Marjolia-Base | 50 |
| Eichenmoos abs. Marokk. entf. 50 % | 30 |
| Patchouliöl entfärbt | 220 |
| 1,3,4,6,7,8-Hexahydro-4,6,6,7,8,8-hexamethyl-cyclopenta-$\gamma$-2-benzopyran 50 % in Diethylphthalat | 100 |
| Orcincarbonsäuremethylester | 10 |
| 2,6,9-Trimethyl-cyclododecatri-2,5,9-en-1-ol + Isomer | 160 |
| Dipropylenglycol | 85 |
| | 1.000 |

## Patentansprüche

**1.** Verbindungen der Formel

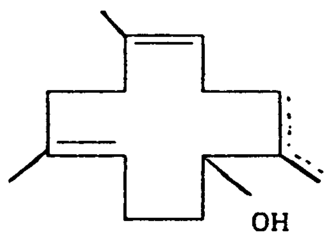

in der
die gestrichelten Linien die beiden möglichen Positionen der dritten Doppelbindung darstellen.

**2.** Verfahren zur Herstellung der Verbindungen nach Anspruch 1, dadurch gekennzeichnet, daß man 1,5,10-Trimethyl-cyclododeca-tri-1,5,9-en in an sich bekannter Weise mit einer Persäure monoepoxidiert und das erhaltene Monoepoxid 1,6,9-Trimethyl-1,2-epoxy-cyclododeca-5,9-dien durch Erhitzen in Gegenwart von Aluminiumisopropylat isomerisiert.

**3.** Verwendung der Verbindungen nach Anspruch 1 als Riechstoff.

## Claims

**1.** Compounds of the formula

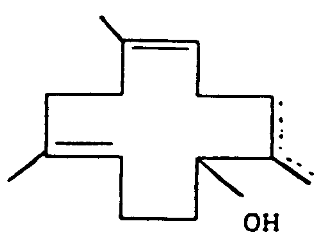

in which
the dashed lines represent the two possible positions of the third double bond.

**2.** Process for the preparation of compounds according to Claim 1, characterised in that 1,5,10-trimethyl-cyclododeca-tri-1,5,9-ene is monoepoxidised in a fashion which is known per se using a peracid, and the monoepoxide obtained, 1,6,9-trimethyl-1,2-epoxycyclododeca-5,9-diene, is isomerised by heating in the presence of aluminium isopropylate.

**3.** Use of compounds according to Claim 1 as scents.

## Revendications

1. Composés de formule

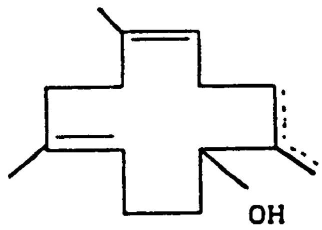

dans laquelle
les traits interrompus représentent les deux positions possibles de la troisième double liaison.

2. Procédé de préparation des composés de la revendication 1, caractérisé en ce que l'on soumet le 1,5,10-triméthylcyclododécatri-1,5,9-ène à monoépoxydation par un peracide de manière connue en soi et on isomérise le monoépoxyde obtenu, 1,6,9-triméthyl-1,2-époxycyclododéca-5,9-diène, par chauffage en présence d'isopropylate d'aluminium.

3. Utilisation des composés de la revendication 1 en tant que matières odorantes.